(19) Europäisches Patentamt
European Patent Office
Office européen des brevets

(11) **EP 3 773 447 B1**

(12) **EUROPÄISCHE PATENTSCHRIFT**

(45) Veröffentlichungstag und Bekanntmachung des Hinweises auf die Patenterteilung:
**03.07.2024 Patentblatt 2024/27**

(21) Anmeldenummer: **19716198.7**

(22) Anmeldetag: **12.04.2019**

(51) Internationale Patentklassifikation (IPC):
**A61K 8/37** *(2006.01)* **A61K 8/46** *(2006.01)*
**A61K 8/36** *(2006.01)* **A61K 8/86** *(2006.01)*
**A61Q 5/02** *(2006.01)* **A61Q 19/10** *(2006.01)*

(52) Gemeinsame Patentklassifikation (CPC):
**A61K 8/37; A61K 8/463; A61K 8/86; A61Q 5/02; A61Q 19/10;** A61K 2800/805

(86) Internationale Anmeldenummer:
**PCT/EP2019/059463**

(87) Internationale Veröffentlichungsnummer:
**WO 2019/197626 (17.10.2019 Gazette 2019/42)**

(54) **OPAKE ZUSAMMENSETZUNG ENTHALTEND ETHYLENGLYKOLDISTEARAT**

OPAQUE COMPOSITION CONTAINING ETHYLENE GLYCOL DISTEARATE

COMPOSITION OPAQUE CONTENANT DE DISTÉARATE D'ÉTHYLÈNE GLYCOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.04.2018 EP 18167225**

(43) Veröffentlichungstag der Anmeldung:
**17.02.2021 Patentblatt 2021/07**

(73) Patentinhaber: **Evonik Operations GmbH**
**45128 Essen (DE)**

(72) Erfinder:
• **SCHWAB, Peter**
**45134 Essen (DE)**
• **SCHUCH, Dominik**
**40221 Düsseldorf (DE)**
• **WINTER, Patrick**
**45473 Mülheim an der Ruhr (DE)**
• **MUSS, Peter**
**45359 Essen (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
EP-A1- 0 568 848 DE-C1- 19 801 231
JP-A- 2003 055 165 US-A1- 2004 037 793

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Gebiet der Erfindung

Gegenstand der Erfindung ist ein Verfahren zur Herstellung einer opaken Zusammensetzung unter Einsatz von Ethylenglykoldistearat.

Stand der Technik

Ethylenglykoldistearat (EGDS) wird seit langem zur Erzielung von Perlglanzeffekten in Formulierungen eingesetzt. Für kosmetische Formulierungen wird dieser Pearlizer z.B. unter dem INCI-Namen Glycol Distearate vertrieben.

So beschreibt die CN192816613 die Herstellung von perlglänzendem Ethylenglykoldistearat in einem wässrigen Medium mit einer oberflächenaktiven Substanz unter Erwärmen und Abkühlen und anschließender pH-Wertjustierung. Das perlglänzende Ethylenglykoldistearat wird auch in wasserarmen Autowaschformulierungen eingesetzt.

JP2003055165 offenbart Trübungsmittel aus Fettsäureglykolestern und Tensiden.

Aufgrund der anhaltenden Diskussion über Mikroplastik in kosmetischen Produkten suchen Hersteller nach Alternativen zu den als Trübungsmittel (Opacifier) eingesetzten Styrol-Acrylat Copolymeren (Styrene/Acrylates Copolymer).

Überraschenderweise wurde gefunden, dass ethylenglykoldistearathaltige Zusammensetzungen, hergestellt nach einem neuartigen Verfahren, als alternative Trübungsmittel verwendet werden können. Die in der vorliegenden Erfindung beschriebenen Ethylenglykoldistearat-Zusammensetzungen zeichnen sich in der Regel durch eine verglichen zu herkömmlichen Ethylenglykoldistearat-Zusammensetzungen reduzierten Perlglanzeffekt aus.

Aufgabe der Erfindung war es, Ersatzsubstanzen für auf Styrol-Acrylat Copolymeren basierende Trübungsmittel bereitzustellen.

Beschreibung der Erfindung

Gegenstand der vorliegenden Erfindung ist daher ein Verfahren zur Herstellung einer Zusammensetzung enthaltend Ethylenglykoldistearat wie in Anspruch 1 beschrieben.

Ein weiterer Gegenstand der Erfindung sind bestimme opake Zusammensetzungen enthaltend Ethylenglykoldistearat, die als Trübungsmittel verwendet werden können, sowie opake Formulierungen enthaltend die erfindungsgemäßen opaken Zusammensetzungen.

Ein Vorteil der vorliegenden Erfindung ist es, dass die opaken Zusammensetzungen bei vergleichbarer Einsatzkonzentration in Formulierungen einen vergleichbaren Weißgrad und einen vergleichbaren, bevorzugt verbesserten Trübungswert als die herkömmlichen Styrol-Acrylat Copolymere erzeugen.

Ein weiterer Vorteil der vorliegenden Erfindung ist, dass die opaken Zusammensetzungen nicht hochviskos und somit leicht pumpbar sind.

Noch ein Vorteil der vorliegenden Erfindung ist, dass die Bestandteile der Zusammensetzung leicht biologisch abbaubar sind.

Ein weiterer Vorteil der vorliegenden Erfindung ist, dass die Zusammensetzung in einer bestimmten Ausführungsform frei von Polyethylenglycolbestandteilen ist.

Noch ein Vorteil der vorliegenden Erfindung ist der hohe Weißgrad der Trübungsmittel.

Ein weiterer Vorteil der vorliegenden Erfindung ist, dass der Weißgrad sich bei Lagerung nicht verändert.

Noch ein Vorteil der vorliegenden Erfindung ist die geringe Teilchengröße der Trübungsmittel.

Ein weiterer Vorteil der vorliegenden Erfindung ist die geringe Abrasionswirkung der

Trübungsmittel mit einem einhergehenden guten, weichen Hautgefühl.

Ein weiterer Vorteil der vorliegenden Erfindung ist die gute Verträglichkeit der Trübungsmittel mit anderen Formulierungskomponenten, wodurch eine Agglomeration verhindert wird.

Noch ein Vorteil der vorliegenden Erfindung ist die gute Dispergierbarkeit der Trübungsmittel.

Ein weiterer Vorteil der vorliegenden Erfindung ist die geringe Löslichkeit der Trübungsmittel in vielen Lösungsmitteln, insbesondere in für kosmetische Zwecke geeignete Formulierungen.

Noch ein Vorteil der vorliegenden Erfindung ist die herausragende Opazität, gemessen an Feinheit und Brechungsindex der Trübungsmittel.

Vorliegend beansprucht wird somit ein Verfahren zur Herstellung einer opaken Zusammensetzung, welche vorzugsweise keinen Perlglanz aufweist, umfassend die Schritte

a) Bereitstellen einer Ausgangszusammensetzung umfassend

A) 0,5 Gewichtsteile bis 15 Gewichtsteile, bevorzugt 3 Gewichtsteile bis 15 Gewichtsteile, mindestens eines Tensids,

B) 0,5 Gewichtsteile bis 15 Gewichtsteile, bevorzugt 3 Gewichtsteile bis 15 Gewichtsteile mindestens eines Emulgators,

C) 10 Gewichtsteile bis 30 Gewichtsteile Ethylenglykoldistearat,

D) 20 Gewichtsteile bis 85 Gewichtsteile Wasser,

b) Rühren der Ausgangszusammensetzung bei einer Temperatur in einem Bereich von 60 °C bis 100 °C, bevorzugt von 65 °C bis 95 °C, besonders bevorzugt von 70 °C bis 90 °C,

c) Abkühlen auf eine Temperatur in einem Bereich von 5 °C bis 55 °C, bevorzugt von 10 °C bis 50 °C, besonders bevorzugt von 15 °C bis 45 °C unter Erhalt der opaken Zusammensetzung, dadurch gekennzeichnet, dass Komponente A) das mindestens eine Tensid

ausgewählt ist aus gegebenenfalls alkoxylierten, insbesondere gegebenenfalls ethoxylierten, Sulfosuccinaten, oder eine Mischung aus Natriumlaurylethersulfat und/oder gegebenenfalls ethoxyliertem Sulfosuccinat, mit Betain, darstellt und

Komponente B) der mindestens eine Emulgator ausgewählt ist aus der Gruppe bestehend aus Alkoxylaten sowie Fettsäureestern und Glykosiden.

Die angegebenen Gewichtsteile beziehen sich jeweils auf die Gesamtmenge aller Einzeltenside oder Einzelemulgatoren.

Im Unterschied zu transparenten Materialien werden Materialien, die zwar Licht hindurch lassen, aber wie beim Milchglas keine Gegenstände dahinter erkennbar sind als transluzent oder durchscheinend bezeichnet. Bei lichtundurchlässigen Materialien spricht man hingegen von Opazität. Die Opazität ist ein Maß für die Lichtundurchlässigkeit (Trübung) von Stoffen und ist der Kehrwert der Transluzenz. Die Opazität ist der Kehrwert der Transmission.

Unter dem Begriff "opak" im Zusammenhang mit der vorliegenden Erfindung werden folglich Zusammensetzungen verstanden, die zu einem gewissen Maße trüb sind.

Die Trübung, auch Turbidität (lat. turbidus 'trübe') einer Flüssigkeit wird hervorgerufen durch kleine Partikel, die einen vom Trägerstoff abweichenden Brechungsindex besitzen oder Absorption verursachen. Heutzutage wird die Trübung einer Flüssigkeit optisch ermittelt und mittels elektronischer Auswertung gemessen. Die Wellenlänge der Mess-Strahlung liegt üblicherweise im Infrarotbereich bei 860 nm (nach ISO 7027).

In der Regel unterscheidet man zwei Messverfahren:

Die Schwächung der durchgehenden Lichtstrahlung (Durchlicht), am besten geeignet zur Detektion größerer Trübungen.

Die Seitwärtsstreuung der Lichtstrahlung (Streulicht), am besten geeignet zur Detektion kleiner Trübungen.

Um Trübungen vergleichbar messen zu können, wurde die Trübungsstandardflüssigkeit Formazin geschaffen. Alle Trübungseinheiten beziehen sich auf Verdünnungen dieser Flüssigkeit.

Die gebräuchlichsten Trübungseinheiten sind:

- FAU Formazine Attenuation Units - Durchlichtmessung (Winkel 0°) gemäß den Vorschriften der Norm ISO 7027
- FNU Formazine Nephelometric Units - Streulichtmessung (Winkel 90°) gemäß den Vorschriften der Norm ISO 7027
- FTU Formazine Turbidity Unit - in der Wasseraufbereitung verwendete Einheit Unter dem Begriff "opak" im Zusammenhang mit der vorliegenden Erfindung werden folglich Zusammensetzungen verstanden, die einen Trübungswert von 500 oder mehr Formazine Nephelometric Units aufweisen. Die als FNU abgekürzten Formazine Nephelometric Units werden im Zusammenhang mit der vorliegenden Erfindung in einer Streulichtmessung, wie in den Beispielen der vorliegenden Schrift durchgeführt, ermittelt.

Unter dem Begriff "nicht Perlglanz aufweisend" bzw. "nicht Perlglanz-Eigenschaften besitzend" wird im Zusammenhang mit der vorliegenden Erfindung verstanden, dass die betrachtete erfindungsgemäße opake Zusammensetzung als auf eine 3 gewichtsprozentige verdünnte wässrige Zusammensetzung, wobei sich die Gewichtsprozente auf die gesamte wässrige Zusammensetzung beziehen, eine Luminanz von größer oder gleich 187 aufweist. Solch hohe Luminanzwerte können von perlglänzende Zusammensetzungen aufgrund von auftretenden Lichtstreuungen nicht erreicht werden.

Die Luminanz wird im Zusammenhang mit der vorliegenden Erfindung wie in den Beispielen der vorliegenden Schrift durchgeführt ermittelt.

Unter den Begriffen "Tensid" und "Emulgator" werden im Zusammenhang mit der vorliegenden Erfindung organische Substanzen mit grenzflächenaktiven Eigenschaften verstanden, die die Fähigkeit besitzen, die Oberflächenspannung von Wasser bei 20°C und bei einer Konzentration von 0,5 Gew.-% bezogen auf die Gesamtzusammensetzung auf unter 45 mN/m zu verringern. Dabei weisen Tenside positiv und/oder negativ geladene funktionelle Gruppen in ihrer chemischen Strukturformel auf, wohingegen Emulgatoren hinsichtlich ihrer chemischen Strukturformel keine geladen funktionellen Gruppen enthalten, betrachtet in einem pH-Bereich von 2 bis 12 und bei einer Temperatur von 20 °C.

Die Oberflächenspannung wird durch die Ringmethode nach DuNoüy bei 20°C bestimmt.

Folglich können im Zusammenhang mit der vorliegenden Erfindung Emulgatoren und Tenside eindeutig voneinander getrennt werden und überlappen nicht.

Der "pH-Wert" im Zusammenhang mit der vorliegenden Erfindung ist definiert als der Wert, welcher für die entsprechende Zusammensetzung bei 20 °C nach fünf Minuten Rühren mit einer gemäß ISO 4319 (1977) kalibrierten pH-Elektrode gemessen wird.

Alle angegebenen Prozent (%) sind, wenn nicht anders angegeben, Massenprozent.

Es ist in dem erfindungsgemäßen Verfahren bevorzugt, dass die Komponenten A), B), C) und D) in Summe bezogen auf die durch das erfindungsgemäße Verfahren erhaltene Zusammensetzung mindestens 66 Gew.-%, bevorzugt mindestens 90 Gew.-%, ausmachen.

In dem Verfahren der vorliegenden Erfindung ist in Komponente A) das mindestens eine Tensid ausgewählt aus gegebenenfalls alkoxylierte, insbesondere gegebenenfalls ethoxylierte, Sulfosuccinate, besonders bevorzugt von Monoestern-abgeleitete Sulfosuccinate, insbesondere Disodium Laureth Sulfosuccinate oder Disodium Lauryl Sulfosuccinate, oder eine Mischung aus Natriumlaurylethersulfat und/oder gegebenenfalls ethoxyliertem Sulfosuccinat, mit Betain, darstellt.

Dies hat den technischen Effekt, dass Zusammensetzungen mit einer niedrigen Viskosität und gleichzeitig einem hohen Trübungsgrad erhalten werden. Diese Zusammensetzungen sind somit gut pumpbar und können damit leichter verarbeitet werden. In Formulierungen können hohe Trübungsgrade unter Vermeidung von Perlglanz-Effekten erzeugt werden.

Es ist insbesondere von Vorteil, wenn in dem Verfahren der vorliegenden Erfindung Komponente A) eine Mischung aus Natriumlaurylethersulfat und/oder gegebenenfalls ethoxyliertem Sulfosuccinat, insbesondere Natriumlaurylethersulfat, mit Betain, insbesondere Cocamidopropylbetain, bevorzugt in einem Gewichtsverhältnis von Natriumlaurylethersulfat und/oder gegebenenfalls ethoxyliertem Sulfosuccinat zu Betain von 15 zu 1 bis 1 zu 2, darstellt.

Das erfindungsgemässe Verfahren ist dadurch gekennzeichnet, dass in Komponente B) der mindestens eine Emulgator ausgewählt ist aus der Gruppe bestehend aus Alkoxylaten sowie Fettsäureestern und Glykosiden, besonders bevorzugt alkoxylierte, insbesondere ethoxylierte, Fettalkohole, Methylglucosefettsäureester, Sorbitanfettsäureester, Glycerinfettsäureester und Polyglycerinfettsäureester oder Mischestern daraus.

Bevorzugte ethoxylierte Fettalkohole sind ausgewählt aus ethoxylierten Fettalkoholen mit einer Kettenlänge von 8 bis 16 Kohlenstoffatomen, insbesondere ethoxylierter Laurylalkohol, insbesondere Laureth-4.

Bevorzugte Glycerinfettsäureester sind ausgewählt aus Glycerinestern von Fettsäuren ausgewählt aus Fettsäuren mit einer Kettenlänge von 8 bis 22 Kohlenstoffatomen, insbesondere mit einem Veresterungsgrad von im Mittel 0,7 bis 1,5 Fettsäureresten pro Glycerinfettsäureester. Insbesondere sind solche Glycerinfettsäureester ausgewählt aus Glyceryllaurat und Glyceryloleat. Bevorzugte Polyglycerinfettsäureester sind ausgewählt aus solchen mit einem mittleren Polymerisationsgrad des Polyglycerins von 2,5 bis 10, mit einem Veresterungsgrad von im Mittel 0,5 bis 2,0 Fettsäureresten pro Polyglycerinfettsäureester und mit einer Fettsäure ausgewählt aus Fettsäuren mit einer Kettenlänge von 8 bis 22 Kohlenstoffatomen.

Der Polymerisationsgrad n lässt sich dadurch bestimmen, dass die Hydroxylzahl des zur Synthese des erfindungsgemäßen Esters eingesetzten Polyglycerins ermittelt wird, wobei der mittlere Polymerisationsgrad n mit der Hydroxylzahl des zugrundeliegenden Polyglycerins über folgende Gleichung verknüpft ist:

$$n = \frac{\dfrac{2000 \bullet M(KOH)}{OHZ} - M(Wasser)}{\left[ M(Glycerin) - M(Wasser) \right] - \dfrac{1000 \bullet M(KOH)}{OHZ}}$$

mit M = molare Masse; OHZ = Hydroxylzahl des freien Polyglycerins.

Alternativ lässt sich der Polymerisationsgrad n auch dadurch bestimmen, dass die Hydroxylzahl des nach vollständiger Esterhydrolyse erhaltenen Polyglycerins ermittelt wird.

Geeignete Bestimmungsmethoden zur Ermittlung der Hydroxylzahl sind insbesondere solche gemäß DGF C-V 17 a (53), Ph. Eur. 2.5.3 Method A und DIN 53240.

In einem erfindungsgemäß besonders bevorzugten Verfahren ist Komponente A) ausgewählt aus einer Mischung aus Natriumlaurylethersulfat mit Betain, bevorzugt in einem Gewichtsverhältnis von 12 zu 1 bis 1 zu 1, und Komponente B) ausgewählt aus Glycerinfettsäureestern, insbesondere Glyceryloleat. Dies hat den technischen Effekt, dass Zusammensetzungen mit einer besonders guten Lagerstabilität und einem sehr hohen Trübungsgrad erhalten werden, die in Formulierungen keinen Perlglanz-Effekt erzeugen.

In einem erfindungsgemäß alternativ besonders bevorzugten Verfahren ist Komponente A) ausgewählt aus ethoxyliertem Sulfosuccinat und Komponente B) ausgewählt aus ethoxylierten Fettalkoholen, insbesondere ethoxylierter Laurylalkohol, insbesondere Laureth-4.

In einer bevorzugten Ausführungsform ist das erfindungsgemäße Verfahren eines zur Herstellung einer opaken Zusammensetzung, welche im Wesentlichen frei von Polyethern und Polyether enthaltenden Verbindungen ist und vorzugsweise keinen Perlglanz aufweist.

Somit sind alle in dieser bevorzugten Ausführungsform des erfindungsgemäßen Verfahrens eingesetzten Komponenten im Wesentlichen frei von Polyethern und Polyether enthaltenden Verbindungen.

Der Begriff "im Wesentlichen frei von Polyethern und Polyether enthaltenden Verbindungen" beschreibt im Zusammenhang mit der vorliegenden Erfindung, dass enthaltene Verbindungen nur in Spuren, bevorzugt keine, Alkoxygruppen, Oligoalkoxygruppen oder Polyalkoxygruppen wie z.B. Ethylenoxid oder Propylenoxid enthalten. Die Konzentration an Polyether enthaltenden Verbindungen sollte kleiner 0,1 Gew.-%, insbesondere bevorzugt kleiner 0,01 Gew.-% bezogen auf die Gesamtformulierung, bevorzugt unterhalb der Nachweisgrenze gängiger Analyseverfahren wie beispielsweise GC, HPLC, NMR-Spektroskopie, GPC oder Maldi sein.

In dieser bevorzugten Ausführungsform des erfindungsgemäßen Verfahrens ist bevorzugt eingesetzte Komponente A) ausgewählt aus Sulfosuccinaten, bevorzugt aus von Monoestern-abgeleitete Sulfosuccinaten, wie beispielsweise SulfosuccinateDisodium Lauryl Sulfosuccinate, Disodium Undecylenamido MEA Sulfosuccinate, Disodium Ricinoleamido MEA Sulfosuccinate, Dietylhexyl Sodium Sulfosuccinate, Disodium Cocamide MEA Sulfosuccinate, Ammonium Dinonyl Sulfosuccinate, Ammonium Lauryl Sulfosuccinate, Diammonium Lauramido MEA Sulfosuccinate, Diammonium Lauryl Sulfosuccinate, Diamyl Sodium Sulfosuccinate, Dicapryl Sodium Sulfosuccinate, Dicyclohexyl Sodium Sulfosuccinate, Diheptyl Sodium Sulfosuccinate, Dihexyl Sodium Sulfosuccinate, Diisobutyl Sodium Sulfosuccinate, Dioctyl Sodium Sulfosuccinate, Disodium Cetearyl Sulfosuccinate, Disodium Cocamide MEA-Sulfosuccinate, Cocamide MIPA-Sulfosuccinate Disodium, Disodium Coco-Glucoside Sulfosuccinate, Disodium Dihydroxietyl Sulfosuccinyl Undecilenate, Disodium Hydrogenated Cottonseed Glyceride Sulfosuccinate, Disodium Isodecyl Sulfosuccinate, Disodium Isostearamido MEA-Sulfosuccinate, Disodium Isostearamido MIPA-Sulfosuccinate, Disodium Isostearyl Sulfosuccinate, Disodium Lauramido MEA-Sulfosuccinate, Disodium Myristamido MEA-Sulfosuccinate, Disodium Oleamido MEA Sulfosuccinate-, Disodium Oleamido MIPA Sulfosuccinate, Disodium Oleyl Sulfosuccinate, Disodium Ricinoleamido MEA-Sulfosuccinate, Disodium Stearamido MEA-Sulfosuccinate, Disodium Stearyl Sulfosuccinamate, Disodium Stearyl Sulfosuccinate, Disodium Tallamido MEA-Sulfosuccinate, Disodium Tallowamido MEA-Sulfosuccinate, Disodium Tallow Sulfosuccinamate, Disodium Tridecyl Sulfosuccinate, Disodium Wheat germamido MEA-Sulfosuccinate, Ditridecyl Sodium Sulfosuccinate, Sodium Bisglycol RicinoSulfosuccinate und Komponente B) ist ausgewählt aus Methylglucosefettsäureester, Sorbitanfettsäureester, Glycerinfettsäureester und Polyglycerinfettsäureester und Mischestern der vorgenannten , wobei die oben bereits als bevorzugt genannten Vertreter der Glycerinfettsäureester besonders bevorzugt sind.

Als Komponente C) kann als Ethylenglykoldistearat auch technisches Ethylenglykoldistearat eingesetzt werden, dessen Veresterungsgrad beispielsweise nicht exakt bei zwei ("Di-Ester") liegt, sondern bei beispielsweise 1,5 (sogenanntes "Ethylenglykolsesquistearat"). Zusätzlich oder alternativ kann beispielsweise ein Teil der Stearinsäurereste durch Palmitinsäurereste substituiert sein.

Es ist erfindungsgemäß bevorzugt, dass die Ausgangszusammensetzung des Verfahrensschrittes a) bei 20 °C einen pH-Wert von 4 bis 7 aufweist.

Es ist erfindungsgemäß bevorzugt, dass das Rühren in Verfahrensschritt b) von 0,25 bis 6,0 Stunden, bevorzugt von 0,5 bis 4,0 Stunden dauert.

Ein erfindungsgemäß bevorzugtes Verfahren ist dadurch gekennzeichnet, dass in Verfahrensschritt b) die Komponenten A) bis D) der Ausgangszusammensetzung in Form einer homogenen Emulsion vorliegen.

Verfahrensschritt c) des erfindungsgemäßen Verfahrens wird bevorzugt mit einer Abkühlungsgeschwindigkeit von 0,1 °C/min bis 15 °C/min, bevorzugt von 0,2 °C/min bis 10 °C/min, besonders bevorzugt von 0,3 °C/min bis 5 °C/min, durchgeführt.

In den Verfahrensschritten b) und/oder c) kann zusätzlich Komponente D), Wasser, hinzugefügt werden, bevorzugt bis zu einer maximalen Konzentration von 85 Gew.-%, bezogen auf die opake Zusammensetzung.

Ein erfindungsgemäß bevorzugtes Verfahren ist dadurch gekennzeichnet, dass in Verfahrensschritt c) das Abkühlen teilweise durch den Zusatz von Wasser zu der Ausgangszusammensetzung durchgeführt wird, bevorzugt unter Erhalt einer opaken Zusammensetzung mit einem Wassergehalt von 50 Gew.-% bis 85 Gew.-% Wasser, wobei sich die Gewichtsprozente auf die Gesamtzusammensetzung beziehen.

Ein weiterer Gegenstand der vorliegenden Erfindung ist eine opake Zusammensetzung erhältlich durch das erfindungsgemäße Verfahren.

Noch ein weiterer Gegenstand der vorliegenden Erfindung ist eine opake Zusammensetzung enthaltend

A2) 0,5 Gew.-% bis 15 Gew.-%, bevorzugt 3 Gew.-% bis 15 Gew.-%, mindestens eines Tensids,
B2) 0,5 Gew.-% bis 15 Gew.-%, bevorzugt 3 Gew.-% bis 15 Gew.-% mindestens eines Emulgators,

C2) 10 Gew.-% bis 30 Gew.-% Ethylenglykoldistearat,
D2) 50 Gew.-% bis 84 Gew.-% Wasser,

wobei sich die Gewichtsprozente auf die Gesamtzusammensetzung beziehen, dadurch gekennzeichnet, dass Komponente A2) das mindestens eine Tensid ausgewählt ist aus gegebenenfalls alkoxylierten, insbesondere gegebenenfalls ethoxylierten, Sulfosuccinaten, oder eine Mischung aus Natriumlaurylethersulfat und/oder gegebenenfalls ethoxyliertem Sulfosuccinat, mit Betain, darstellt und Komponente B2) der mindestens eine Emulgator ausgewählt ist aus der Gruppe bestehend aus Alkoxylaten sowie Fettsäureestern und Glykosiden.

Erfindungsgemäß bevorzugt enthalten die erfindungsgemäßen Zusammensetzungen die Komponenten A2), B2, C2) und D2) in Summe zu mindestens 66 Gew.-%, bevorzugt mindestens 90 Gew.-%, bezogen auf die Gesamtzusammensetzung.

Bevorzugt weisen die opaken Zusammensetzungen erhältlich durch das erfindungsgemäße Verfahren und die erfindungsgemäßen Zusammensetzungen eine Viskosität von 10 bis 30000 mPa s auf, besonders bevorzugt eine Viskosität von 100 bis 10000 mPa s, ganz besonders bevorzugt eine Viskosität von 500 bis 7000 mPa s gemessen bei 25 °C mit Brookfield Viskosimeter mit RV Spindel = 5 und bei 10 rpm.

Bevorzugt weisen die opaken Zusammensetzungen erhältlich durch das erfindungsgemäße Verfahren und die erfindungsgemäßen opaken Zusammensetzungen bei einer Konzentration von 0,3 Gew.-% in Wasser, wobei sich die Gewichtsprozente auf die Summe von Wasser und Gesamtzusammensetzung beziehen, einen Trübungswert von 500, bevorzugt 1000, besonders bevorzugt 1200, oder mehr Formazine Nephelometric Units auf.

Die opaken Zusammensetzungen erhältlich durch das erfindungsgemäße Verfahren und die erfindungsgemäßen opaken Zusammensetzungen unterscheiden sich insbesondere von denen des Standes der Technik dadurch, dass sie keine Perlglanz-Eigenschaften besitzen.

Bevorzugt weisen die opaken Zusammensetzungen erhältlich durch das erfindungsgemäße Verfahren und die erfindungsgemäßen opaken Zusammensetzungen als auf eine 3 gewichtsprozentige verdünnte wässrige Zusammensetzung, wobei sich die Gewichtsprozente auf die gesamte wässrige Zusammensetzung beziehen, eine Luminanz von größer 187, bevorzugt größer 190 auf.

Die Komponenten A2), B2), C2) und D2) sind entsprechend der Gewichtung der Bevorzugung der jeweiligen Komponenten A), B), C) und D) des erfindungsgemäßen Verfahrens bevorzugt; Analoges gilt für entsprechend bevorzugte Kombinationen der Komponenten.

Die opaken Zusammensetzungen erhältlich durch das erfindungsgemäße Verfahren und die erfindungsgemäßen opaken Zusammensetzungen sind somit eben auch bevorzugt im Wesentlichen frei von Polyethern und Polyether enthaltenden Verbindungen.

Die opaken Zusammensetzungen erhältlich durch das erfindungsgemäße Verfahren und die erfindungsgemäßen opaken Zusammensetzungen sind hervorragend zur Herstellung von opaken Formulierungen, insbesondere kosmetische Formulierungen, ganz besonders von Reinigungsformulierungen für Haut und/oder Haare, geeignet.

Diese erfindungsgemäßen opaken Zusammensetzungen lassen sich in einem weiteren erfindungsgemäßen Verfahren zur Herstellung von opaken Formulierungen, insbesondere von opaken kosmetischen Formulierungen, ganz besonders von opaken Reinigungsformulierungen für Haut und/oder Haare, herstellen, welches die Verfahrensschritte umfasst:

a) Bereitstellen einer Ausgangszusammensetzung umfassend
A) 0,5 Gewichtsteile bis 15 Gewichtsteile, bevorzugt 3 Gewichtsteile bis 15 Gewichtsteile, mindestens eines Tensids,
B) 0,5 Gewichtsteile bis 15 Gewichtsteile, bevorzugt 3 Gewichtsteile bis 15 Gewichtsteile mindestens eines Emulgators,
C) 10 Gewichtsteile bis 30 Gewichtsteile Ethylenglykoldistearat,
D) 20 Gewichtsteile bis 85 Gewichtsteile Wasser,
b) Rühren der Ausgangszusammensetzung bei einer Temperatur in einem Bereich von 60 °C bis 100 °C, bevorzugt von 65 °C bis 95 °C, besonders bevorzugt von 70 °C bis 90 °C,
c) Abkühlen auf eine Temperatur in einem Bereich von 5 °C bis 55 °C, bevorzugt von 10 °C bis 50 °C, besonders bevorzugt von 15 °C bis 45 °C unter Erhalt der opaken Zusammensetzung,
d) Vermengen der opaken Zusammensetzung mit weiteren Komponenten in einem Temperaturbereich von 5 °C bis 40 °C, bevorzugt von 10 °C bis 35 °C, besonders bevorzugt von 15 °C bis 25 °C, unter Erhalt einer opaken Formulierung, dadurch gekennzeichnet, dass Komponente A) das mindestens eine Tensid ausgewählt ist aus gegebenenfalls alkoxylierten, insbesondere gegebenenfalls ethoxylierten, Sulfosuccinaten, oder eine Mischung aus Natriumlaurylethersulfat und/oder gegebenenfalls ethoxyliertem Sulfosuccinat, mit Betain, darstellt und Komponente B) der mindestens eine Emulgator ausgewählt ist aus der Gruppe bestehend aus Alkoxylaten sowie Fettsäureestern und Glykosiden.

In diesem weiteren erfindungsgemäßen Verfahren werden in Verfahrensschritt a) bis c) die gleichen bevorzugten Ausführungsformen eingesetzt, wie oben für das erste erfindungsgemäße Verfahren beschrieben.

Es ist in diesem weiteren erfindungsgemäßen Verfahren bevorzugt, dass die Komponenten A), B), C) und D) in Summe bezogen auf die durch dieses weitere erfindungsgemäße Verfahren erhaltene Formulierung mindestens 0,5 Gew.-% ausmachen.

Ein weiterer Gegenstand der vorliegenden Erfindung sind somit auch opake Formulierungen enthaltend opake Zusammensetzungen erhältlich durch das erste erfindungsgemäße Verfahren und/oder erfindungsgemäße Zusammensetzungen, bevorzugt in einer Menge von 0,1 Gew.-% bis 15 Gew.-%, besonders bevorzugt von 0,5 Gew.-% bis 10 Gew.-%, ganz besonders bevorzugt von 1 Gew.-% bis 6 Gew.-% wobei sich die Gewichtsprozente auf die Gesamtformulierung beziehen.

Bevorzugt weisen die erfindungsgemäßen opaken Formulierungen einen Trübungswert von 600, bevorzugt 750, insbesondere 1000, oder mehr Formazine Nephelometric Units auf.

Die erfindungsgemäßen opaken Formulierungen sind bevorzugt dadurch gekennzeichnet, dass sie keine Perlglanz-Eigenschaften besitzen.

Ein weiterer Gegenstand der vorliegenden Erfindung ist die Verwendung von opaken Zusammensetzungen erhältlich durch das erste erfindungsgemäße Verfahren und/oder der erfindungsgemäßen opaken Zusammensetzungen als Trübungsmittel, insbesondere ohne bei der erfindungsgemäßen Verwendung einen Perlglanz Effekt hervorzurufen.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Abbildungen sind Bestandteil der Beispiele:

Abbildung 1: Trübungswerte der erfindungsgemäßen Formulierungen 1-7 und Vergleichsformulierung V1-V7
Abbildung 2: Trübungswerte der in Wasser verdünnten erfindungsgemäßen opaken Zusammensetzung nach Beispiel 1 und des Marktstandards Styrene/Acrylates Copolymer.

Beispiele:

Im Folgenden wurden die Bestandteile der Zusammensetzungen in Form der allgemein anerkannten INCI-Nomenklatur unter Verwendung der englischen Begriffe benannt. Die Viskositäten wurden mit einem Brookfield-Viskosimeter und einer Spindel 5 bei 22 °C gemessen. Die Umdrehungszahl wurde je nach Widerstand auf 10 oder 100 Umdrehungen pro Minute eingestellt.

*Beispiel 1: Herstellung erfindungsgemäßer opaker Zusammensetzungen*

*Beispiel 1.1: Erfindungsgemäße opake Zusammensetzung*

Eine Mischung aus 5 Gewichtsteilen Disodium Laureth Sulfosuccinate, 7 Gewichtsteilen Laureth-4, 17 Gewichtsteilen Glycol Distearate und 25 Gewichtsteilen Wasser wurde 0,5 h lang bei 80 °C gerührt. Anschließend wurde die Mischung unter Zugabe von 45 Gewichtsteilen Wasser (20 °C) innerhalb von 20 Minuten auf 35 °C abgekühlt.

Es wurde eine weiße, homogene Suspension erhalten, die bei 22 °C gelagert wurde und eine Viskosität von 340 mPa s nach 24 h sowie 452 mPa s nach 1 Woche aufwies. Die Suspension zeigte innerhalb von 6 Wochen keine Separationseffekte.

*Beispiel 1.2: Erfindungsgemäße opake Zusammensetzung*

Eine Mischung aus 6 Gewichtsteilen Sodium Laureth Sulfate, 7 Gewichtsteilen Laureth-4, 16 Gewichtsteilen Glycol Distearate und 37 Gewichtsteilen Wasser wurde 0,5 h lang bei 80 °C gerührt. Anschließend wurde unter Zugabe von 34 Gewichtsteilen Wasser (20 °C) innerhalb von 0,5 h auf 35 °C abgekühlt.

Es wurde eine weiße, homogene Suspension erhalten, die bei 22 °C gelagert wurde und eine Viskosität von 11640 mPa s nach 24 h sowie 16400 mPa s nach 1 Woche aufwies. Die Suspension zeigte innerhalb von 6 Wochen keine Separationseffekte.

*Beispiel 1.3: Erfindungsgemäße opake Zusammensetzung*

Eine Mischung aus 9 Gewichtsteilen Sodium Laureth Sulfate, 2 Gewichtsteilen Cocamidopropyl Betaine, 1 Gewichtsteil Glyceryl Oleate, 25 Gewichtsteilen Glycol Distearate und 63 Gewichtsteilen Wasser wurde 0,5 h lang bei 80 °C gerührt. Anschließend wurde innerhalb von 1,5 h auf 27 °C abgekühlt.

Es wurde eine weiße, homogene Suspension erhalten, die bei 22 °C gelagert wurde und eine Viskosität von 2600 mPa s nach 24 h sowie 3700 mPa s nach 1 Woche aufwies. Die Suspension zeigte innerhalb von 6 Wochen keine Separationseffekte.

*Beispiel 1.4: Erfindungsgemäße opake Zusammensetzung*

Eine Mischung aus 6 Gewichtsteilen Disodium Lauryl Sulfosuccinate, 7 Gewichtsteilen Glyceryl Laurate, 17 Gewichtsteilen Glycol Distearate und 24 Gewichtsteilen Wasser wurde 0,5 h lang bei 80 °C gerührt. Anschließend wurde die Mischung unter Zugabe von 46 Gewichtsteilen Wasser (20 °C) innerhalb von 20 Minuten auf 35 °C abgekühlt.

Es wurde eine weiße, homogene Suspension erhalten, die bei 22 °C gelagert wurde und nach 24 h eine Viskosität von 30000 mPa s aufwies. Die Suspension zeigte innerhalb von 6 Wochen keine Separationseffekte.

*Beispiel 1.5: Erfindungsgemäße opake Zusammensetzung*

Eine Mischung aus 6 Gewichtsteilen Disodium Lauryl Sulfosuccinate, 5 Gewichtsteilen Glyceryl Laurate, 17 Gewichtsteilen Glycol Distearate und 39 Gewichtsteilen Wasser wurde 0,5 h lang bei 80 °C gerührt. Anschließend wurde die Mischung unter Zugabe von 33 Gewichtsteilen Wasser (20 °C) innerhalb von 20 Minuten auf 35 °C abgekühlt.

Es wurde eine weiße, homogene Suspension erhalten, die bei 22 °C gelagert wurde und nach 24 h eine Viskosität von 1080 mPa s aufwies. Die Suspension zeigte innerhalb von 6 Wochen keine Separationseffekte.

*Beispiel 1.6: Erfindungsgemäße opake Zusammensetzung*

Eine Mischung aus 6 Gewichtsteilen Disodium Lauryl Sulfosuccinate, 6 Gewichtsteilen Glyceryl Oleate, 17 Gewichtsteilen Glycol Distearate und 39 Gewichtsteilen Wasser wurde 0,5 h lang bei 80 °C gerührt. Anschließend wurde die Mischung unter Zugabe von 32 Gewichtsteilen Wasser (20 °C) innerhalb von 20 Minuten auf 35 °C abgekühlt.

Es wurde eine weiße, homogene Suspension erhalten, die bei 22 °C gelagert wurde und nach 24 h eine Viskosität von 2400 mPa s aufwies. Die Suspension zeigte innerhalb von 6 Wochen keine Separationseffekte.

*Beispiel 2: Erfindungsgemäße opake Zusammensetzung*

Eine Mischung bestehend aus 8 Gew.-% Disodium Cocoamphopropionate, 10 Gew.-% Laureth-4, 25 Gew.-% Glycol Distearate und 57 Gew.-% Wasser wurde 1 h lang bei 80 °C gerührt. Anschließend wurde die Mischung innerhalb von 60 Minuten auf 30 °C abgekühlt.

*Beispiel 3: Bestimmung der Trübungswerte von erfindungsgemäßen Formulierungen enthaltend erfindungsgemäße opake Zusammensetzung*

Die erfindungsgemäße Zusammensetzung aus Beispiel 1.1 wurde nach Tabelle 1 in eine Tensidmischung bestehend aus 9 Gewichtsteilen SLES (Sodium Laureth Sulfate ) und 3 Gewichtsteilen CAPB (Cocamidopropyl-Betaine) homogen eingerührt und durch Zugabe von 1% NaCl verdickt:

Tab. 1: Erfindungsgemäße opake Formulierungen 1-7 hergestellt aus erfindungsgemäßem Beispiel 1.1

|  | 1 | 2 | 3 | 4 | 5 | 6 | 7 |
|---|---|---|---|---|---|---|---|
| **SLES (28% a.m.)** | 32 | 32 | 32 | 32 | 32 | 32 | 32 |
| **Water** | 58,65 | 58,45 | 58,25 | 58,05 | 57,95 | 57,85 | 57,75 |
| **TEGO Betain F 50 (38% a.m.)** | 8 | 8 | 8 | 8 | 8 | 8 | 8 |
| **Erfindungsgemäßes Beispiel 1.1** | 0,1 | 0,3 | 0,5 | 0,7 | 0,8 | 0,9 | 1 |
| **NaCl** | 0,8 | 0,8 | 0,8 | 0,8 | 0,8 | 0,8 | 0,8 |
| **Neolone PE** | 0,45 | 0,45 | 0,45 | 0,45 | 0,45 | 0,45 | 0,45 |
| **pH** | 5,5 | 5,5 | 5,5 | 5,5 | 5,5 | 5,5 | 5,5 |

Zum Vergleich wurde ein Benchmark Styrene/Acrylates Copolymer ebenfalls in die gleiche Tensidmischung homogen eingerührt und analog verdickt:

Tab. 2: Vergleichsformulierungen V1-V7

|  | V1 | V2 | V3 | V4 | V5 | V6 | V7 |
|---|---|---|---|---|---|---|---|
| **SLES (28% a.m.)** | 32 | 32 | 32 | 32 | 32 | 32 | 32 |
| **Water** | 58,65 | 58,45 | 58,25 | 58,05 | 57,95 | 57,85 | 57,75 |
| **TEGO Betain F 50 (38% a.m.)** | 8 | 8 | 8 | 8 | 8 | 8 | 8 |
| **Acusol OP 301 (17% a.m.)** | 0,1 | 0,3 | 0,5 | 0,7 | 0,8 | 0,9 | 1 |
| **NaCl** | 0,8 | 0,8 | 0,8 | 0,8 | 0,8 | 0,8 | 0,8 |
| **Neolone PE** | 0,45 | 0,45 | 0,45 | 0,45 | 0,45 | 0,45 | 0,45 |
| **pH** | 5,5 | 5,5 | 5,5 | 5,5 | 5,5 | 5,5 | 5,5 |

Mit den erfindungsgemäßen opaken Formulierungen 1-7 und den Vergleichsformulierungen V1-V7 wurden die Trübungswerte ermittelt. Das verwendete Meßgerät war ein Iso Turbidity Meter, HI 88713 von Hanna Instruments, welches im Messmodus: "NTU ratio mode" betrieben wurde. Dazu wurden indizierte Küvetten bis zur 10 mL Markierung gefüllt und entgast. Die Küvetten wurden anschließend in der Messzelle bei Raumtemperatur vermessen. Die Küvette wurde nach jeder Messung um 90 ° gedreht. Insgesamt wurde so viermal gemessen und anschließend der Mittelwert errechnet. Die Messwerte werden in NTU ausgewiesen.

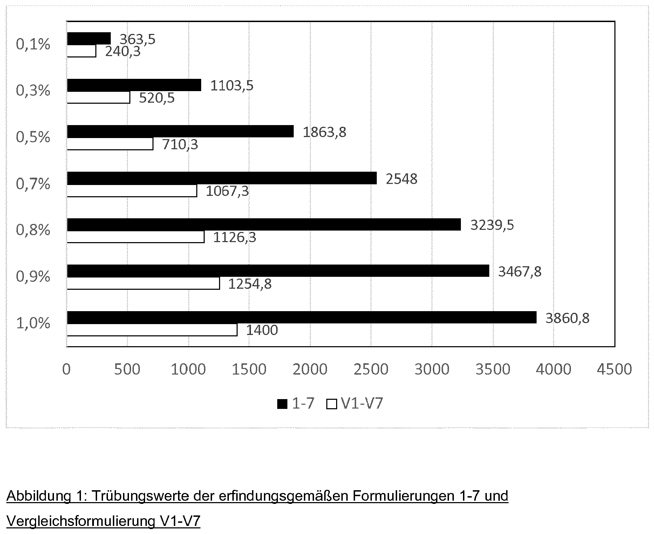

Die Ergebnisse sind in Abbildung 1 dargestellt. Die Messwerte in Abbildung 1 zeigen, dass die erfindungsgemäßen Formulierungen bei gleichem Aktivgehalt signifikant höhere Trübungswerte aufweisen.

*Beispiel 4: Trübungswerte der erfindungsgemäßen Zusammensetzungen*

Analog zu Beispiel 3 wurden die Trübungswerte der erfindungsgemäßen Zusammensetzung nach Beispiel 1.1 in einer Verdünnungsreihe in Wasser gemessen.

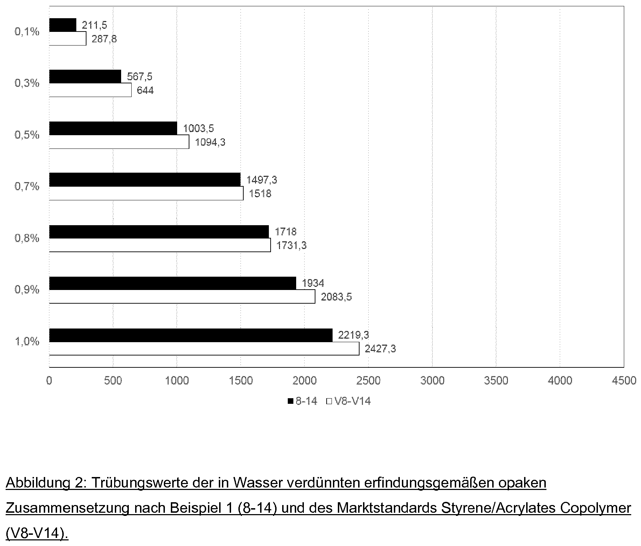

Die Ergebnisse sind in Abbildung 2 zusammengefasst: Die erfindungsgemäßen opaken Zusammensetzung zeigt vergleichbare Trübungswerte wie der Marktstandard Styrene/Acrylates Copolymer.

Analog zu Beispiel 3 wurden die Trübungswerte der erfindungsgemäßen Zusammensetzung nach den Beispielen 1.1 bis 2 in einer Konzentration von 0,5 Gew.-% gemessen.

Trübungswerte 0,5 %ig in SLES/CAPB:

| Beispiel 1.1 | 1864 NTU |
|---|---|
| Beispiel 1.2 | 1739 NTU |
| Beispiel 1.3 | 4000 NTU |
| Beispiel 1.4 | 1628 NTU |
| Beispiel 1.5 | 1660 NTU |
| Beispiel 1.6 | 1669 NTU |
| Beispiel 2 | 1350 NTU |

*Beispiel 5: Bestimmung der Perlglanz-Eigenschaften*

Zur Bestimmung der Luminanz werden 3% der Blends in eine Tensidmischung aus SLES/CAPB (11.2:3.8 active matter) sorgfältig eingerührt, die mit 0,8% PEG-18 Glyceryl Oleate/Cocoate (ANTIL 171, kommerziell vertrieben von EVONIK Nutrition&Care GmbH) und ca. 0,4% NaCl verdickt ist, so dass eine Viskosität von 3000-4000 mPas (Brookfield, Spindel 2, 30 rpm) erreicht wird. 38 g dieser Formulierungen werden anschließend in einen schwarzen Deckel aus Kunststoff (Höhe 1,9 cm; Durchmesser 5,9 cm) eingefüllt und auf einen schwarzen Untergrund platziert, der sich einem Lichtzelt (Hersteller Neewer, Produktnummer 10026118, Größe 40x40x40 cm) befindet. Eine Digitalkamera (Canon EOS 605) bestückt mit einem Zoomobjektiv (Canon EF-S 18-55mm, 1:3.5-5.6) wird 32 cm über der Oberkante des Deckels fixiert. Zur gleichmäßigen Beleuchtung der Probe wird rechts und links des Lichtzeltes jeweils eine externe Lichtquelle aufgestellt (eSmart ESL Photolamp: E27, Durchmessung 72 mm, Länge 235 mm, 50 W, 3200 Lumen, 5500K). Die Fotos werden bei einer Blende von f/5.5 und einer Belichtungszeit von 1/100s, sowie 55 mm Zoomeinstellung aufgenommen. Zur Analyse der Fotos wird das Programm Photoshop CC 2015 Version 2015.0.1 verwendet. Dazu wird ein 60x60mm großer Bildausschnitt aus der Mitte des Fotos ausgeschnitten und das Histogramm ausgelesen. Die so erhaltenen Werte zur Luminanz werden ausgewertet und verglichen.

Als Vergleichssubstanz dient das Perlglanzmittel TEGO Pearl N 300, kommerziell vertrieben von EVONIK Nutrition&Care GmbH.

| | |
|---|---|
| Beispiel 1.1 | 190 |
| Beispiel 1.2 | 187 |
| Beispiel 1.3 | 195 |
| Beispiel 1.4 | 197 |
| Beispiel 1.5 | 187 |
| Beispiel 1.6 | 187 |
| TEGO Pearl N 300 (Vergleich) | 180 |

**Patentansprüche**

1. Verfahren zur Herstellung einer opaken Zusammensetzung umfassend die Schritte

    a) Bereitstellen einer Ausgangszusammensetzung umfassend

        A) 0,5 Gewichtsteile bis 15 Gewichtsteile mindestens eines Tensids,
        B) 0,5 Gewichtsteile bis 15 Gewichtsteile mindestens eines Emulgators,
        C) 10 Gewichtsteile bis 30 Gewichtsteile Ethylenglykoldistearat,
        D) 20 Gewichtsteile bis 85 Gewichtsteile Wasser,

    b) Rühren der Ausgangszusammensetzung bei einer Temperatur in einem Bereich von 60 °C bis 100 °C, bevorzugt von 65 °C bis 95 °C, besonders bevorzugt von 70 °C bis 90 °C,
    c) Abkühlen auf eine Temperatur in einem Bereich von 5 °C bis 55 °C, bevorzugt von 10 °C bis 50 °C, besonders bevorzugt von 15 °C bis 45 °C unter Erhalt der opaken Zusammensetzung, **dadurch gekennzeichnet, dass** Komponente A) das mindestens eine Tensid

        ausgewählt ist aus gegebenenfalls alkoxylierten, insbesondere gegebenenfalls ethoxylierten, Sulfosuccinaten, oder
        eine Mischung aus Natriumlaurylethersulfat und/oder gegebenenfalls ethoxyliertem Sulfosuccinat, mit Betain, darstellt und

    Komponente B) der mindestens eine Emulgator ausgewählt ist aus der Gruppe bestehend aus Alkoxylaten sowie Fettsäureestern und Glykosiden.

2. Verfahren gemäß Anspruch 1, **dadurch gekennzeichnet, dass** in Komponente A) das mindestens eine Tensid ausgewählt ist aus der Gruppe bestehend aus von Monoestern-abgeleitete Sulfosuccinate, insbesondere Disodium Laureth Sulfosuccinate.

3. Verfahren gemäß Anspruch 1 oder 2, **dadurch gekennzeichnet, dass** in Komponente B) der mindestens eine Emulgator ausgewählt ist aus der Gruppe bestehend aus ethoxylierte Fettalkohole sowie Polyglycerinfettsäureester.

4. Verfahren gemäß mindestens einem der vorherigen Ansprüche, **dadurch gekennzeichnet, dass** die Ausgangszusammensetzung des Verfahrensschrittes a) bei 20 °C einen pH-Wert von 4 bis 7 aufweist.

5. Verfahren gemäß mindestens einem der vorherigen Ansprüche, **dadurch gekennzeichnet, dass** Verfahrensschritt c) mit einer Abkühlungsgeschwindigkeit von 0,1 °C/min bis 15 °C/min, bevorzugt von 0,2 °C/min bis 10 °C/min, besonders bevorzugt von 0,3 °C/min bis 5 °C/min, durchgeführt wird.

**6.** Verfahren gemäß mindestens einem der vorherigen Ansprüche, **dadurch gekennzeichnet, dass** in Verfahrensschritt c) das Abkühlen mindestens teilweise durch den Zusatz von Wasser zu der Ausgangszusammensetzung durchgeführt wird, bevorzugt unter Erhalt einer opaken Zusammensetzung mit einem Wassergehalt von 50 Gew.-% bis 85 Gew.-% Wasser, wobei sich die Gewichtsprozente auf die Gesamtzusammensetzung beziehen.

**7.** Opake Zusammensetzung erhältlich durch ein Verfahren gemäß mindestens einem der vorherigen Ansprüche.

**8.** Opake Zusammensetzung enthaltend

A2) 0,5 Gew.-% bis 15 Gew.-% mindestens eines Tensids,
B2) 0,5 Gew.-% bis 15 Gew.-% mindestens eines Emulgators,
C2) 10 Gew.-% bis 30 Gew.-% Ethylenglykoldistearat,
D2) 50 Gew.-% bis 84 Gew.-% Wasser,
wobei sich die Gewichtsprozente auf die Gesamtzusammensetzung beziehen, **dadurch gekennzeichnet, dass** Komponente A2) das mindestens eine Tensid

ausgewählt ist aus gegebenenfalls alkoxylierten, insbesondere gegebenenfalls ethoxylierten, Sulfosuccinaten, oder
eine Mischung aus Natriumlaurylethersulfat und/oder gegebenenfalls ethoxyliertem Sulfosuccinat, mit Betain, darstellt und

Komponente B2) der mindestens eine Emulgator ausgewählt ist aus der Gruppe bestehend aus Alkoxylaten sowie Fettsäureestern und Glykosiden.

**9.** Opake Zusammensetzung nach Anspruch 7 oder 8, **dadurch gekennzeichnet, dass** sie bei einer Konzentration von 0,3 Gew.-% in Wasser, wobei sich die Gewichtsprozente auf die Summe von Wasser und Gesamtformulierung beziehen, einen Trübungswert von 500 oder mehr Formazine Nephelometric Units aufweist.

**10.** Verfahren zur Herstellung von opaken Formulierungen umfassend die Verfahrensschritte

a) Bereitstellen einer Ausgangszusammensetzung umfassend

A) 0,5 Gewichtsteile bis 15 Gewichtsteile mindestens eines Tensids,
B) 0,5 Gewichtsteile bis 15 Gewichtsteile mindestens eines Emulgators,
C) 10 Gewichtsteile bis 30 Gewichtsteile Ethylenglykoldistearat,
D) 20 Gewichtsteile bis 85 Gewichtsteile Wasser,

b) Rühren der Ausgangszusammensetzung bei einer Temperatur in einem Bereich von 60 °C bis 100 °C, bevorzugt von 65 °C bis 95 °C, besonders bevorzugt von 70 °C bis 90 °C,
c) Abkühlen auf eine Temperatur in einem Bereich von 5 °C bis 55 °C, bevorzugt von 10 °C bis 50 °C, besonders bevorzugt von 15 °C bis 45 °C unter Erhalt der opaken Zusammensetzung,
d) Vermengen der opaken Zusammensetzung mit weiteren Komponenten in einem Temperaturbereich von 5 °C bis 40 °C, bevorzugt von 10 °C bis 35 °C, besonders bevorzugt von 15 °C bis 25 °C, unter Erhalt einer opaken Formulierungen, **dadurch gekennzeichnet, dass**
Komponente A) das mindestens eine Tensid

ausgewählt ist aus gegebenenfalls alkoxylierten, insbesondere gegebenenfalls ethoxylierten, Sulfosuccinaten, oder
eine Mischung aus Natriumlaurylethersulfat und/oder gegebenenfalls ethoxyliertem Sulfosuccinat, mit Betain, darstellt und

Komponente B) der mindestens eine Emulgator ausgewählt ist aus der Gruppe bestehend aus Alkoxylaten sowie Fettsäureestern und Glykosiden.

**11.** Opake Formulierung erhältlich durch ein Verfahren gemäß Anspruch 10.

**12.** Opake Formulierung enthaltend eine opake Zusammensetzung gemäß mindestens einem der Ansprüche 7 bis 9, bevorzugt in einer Menge von 0,1 Gew.-% bis 15 Gew.-%, wobei sich die Gewichtsprozente auf die Gesamtzusammensetzung beziehen.

13. Opake Formulierung nach Anspruch 11 oder 12, **dadurch gekennzeichnet, dass** sie einen Trübungswert von 600 oder mehr Formazine Nephelometric Units aufweist.

14. Opake Formulierung gemäß mindestens einem der Ansprüche 11 bis 13, **dadurch gekennzeichnet, dass** sie keine Perlglanz-Eigenschaften besitzt.

15. Verwendung einer opaken Zusammensetzung gemäß mindestens einem der Ansprüche 7 bis 9 als Trübungsmittel.

**Claims**

1. Process for preparing an opaque composition comprising the steps of:

   a) providing a starting composition comprising

   A) 0.5 part by weight to 15 parts by weight of at least one surfactant,
   B) 0.5 part by weight to 15 parts by weight of at least one emulsifier,
   C) 10 parts by weight to 30 parts by weight of ethylene glycol distearate,
   D) 20 parts by weight to 85 parts by weight of water,

   b) stirring the starting composition at a temperature in a range from 60°C to 100°C, preferably 65°C to 95°C and particularly preferably 70°C to 90°C,
   c) cooling to a temperature in a range from 5°C to 55°C, preferably 10°C to 50°C, particularly preferably 15°C to 45°C to obtain the opaque composition, **characterized in that**
   component A), the at least one surfactant,
   is selected from optionally alkoxylated, in particular optionally ethoxylated, sulfosuccinates, or
   is a mixture of sodium lauryl ether sulfate and/or optionally ethoxylated sulfosuccinate with betaine, and component B), the at least one emulsifier, is selected from the group consisting of alkoxylates and fatty acid esters and glycosides.

2. Process according to Claim 1, **characterized in that**, in component A), the at least one surfactant is selected from the group consisting of sulfosuccinates derived from monoesters, especially disodium laureth sulfosuccinate.

3. Process according to Claim 1 or 2, **characterized in that** the at least one emulsifier in component B) is selected from the group consisting of ethoxylated fatty alcohols and polyglycerol fatty acid esters.

4. Process according to at least one of the preceding claims, **characterized in that** the starting composition of process step a) has a pH of 4 to 7 at 20°C.

5. Process according to at least one of the preceding claims, **characterized in that** process step c) is carried out at a cooling rate of 0.1°C/min to 15°C/min, preferably 0.2°C/min to 10°C/min, particularly preferably 0.3°C/min to 5°C/min.

6. Process according to at least one of the preceding claims, **characterized in that** in process step c) the cooling is carried out at least partially by adding water to the starting composition, preferably obtaining an opaque composition having a water content of 50% by weight to 85% by weight water, where the percentages by weight refer to the total composition.

7. Opaque composition obtainable by a process according to at least one of the preceding claims.

8. Opaque composition comprising

   A2) 0.5% by weight to 15% by weight of at least one surfactant,
   B2) 0.5% by weight to 15% by weight of at least one emulsifier,
   C2) 10% by weight to 30% by weight of ethylene glycol distearate,
   D2) 50% by weight to 84% by weight of water,

where the percentages by weight are based on the overall composition, **characterized in that** component A2), the at least one surfactant,

is selected from optionally alkoxylated, in particular optionally ethoxylated, sulfosuccinates, or

is a mixture of sodium lauryl ether sulfate and/or optionally ethoxylated sulfosuccinate with betaine, and component B2), the at least one emulsifier, is selected from the group consisting of alkoxylates and fatty acid esters and glycosides.

9. Opaque composition according to Claim 7 or 8, **characterized in that** said composition, at a concentration of 0.3% by weight in water, where the percentages by weight refer to the sum total of water and total formulation, has a turbidity value of 500 or more formazine nephelometric units.

10. Process for preparing opaque formulations comprising the process steps of

   a) providing a starting composition comprising

   A) 0.5 part by weight to 15 parts by weight of at least one surfactant,
   B) 0.5 part by weight to 15 parts by weight of at least one emulsifier,
   C) 10 parts by weight to 30 parts by weight of ethylene glycol distearate,
   D) 20 parts by weight to 85 parts by weight of water,

   b) stirring the starting composition at a temperature in a range from 60°C to 100°C, preferably 65°C to 95°C and particularly preferably 70°C to 90°C,
   c) cooling to a temperature in a range from 5°C to 55°C, preferably 10°C to 50°C, particularly preferably 15°C to 45°C to obtain the opaque composition,
   d) blending the opaque composition with further components in a temperature range from 5°C to 40°C, preferably 10°C to 35°C, particularly preferably 15°C to 25°C, to obtain an opaque formulation, **characterized in that** component A), the at least one surfactant,
   is selected from optionally alkoxylated, in particular optionally ethoxylated, sulfosuccinates, or
   is a mixture of sodium lauryl ether sulfate and/or optionally ethoxylated sulfosuccinate with betaine, and component B), the at least one emulsifier, is selected from the group consisting of alkoxylates and fatty acid esters and glycosides.

11. Opaque formulation obtainable by a process according to Claim 10.

12. Opaque formulation comprising an opaque composition according to at least one of Claims 7 to 9, preferably in an amount of 0.1% by weight to 15% by weight, where the percentages by weight refer to the total composition.

13. Opaque formulation according to Claim 11 or 12, **characterized in that** said formulation has a turbidity value of 600 or more formazine nephelometric units.

14. Opaque formulation according to at least one of Claims 11 to 13, **characterized in that** said formulation has no pearlescent properties.

15. Use of an opaque composition according to at least one of Claims 7 to 9 as opacifier.

**Revendications**

1. Procédé pour la préparation d'une composition opaque, comprenant les étapes

   a) fourniture d'une composition de départ comprenant

   A) 0,5 partie en poids à 15 parties en poids d'au moins un tensioactif,
   B) 0,5 partie en poids à 15 parties en poids d'au moins un émulsifiant,
   C) 10 parties en poids à 30 parties en poids de distéarate d'éthylèneglycol,
   D) 20 parties en poids à 85 parties en poids d'eau,

   b) agitation de la composition de départ à une températue dans une plage de 60 °C à 100 °C, de préférence de 65 °C à 95 °C, de façon particulièrement préférée de 70 °C à 90 °C,
c) refroidissement jusqu'à une température dans une plage de 5 °C à 55 °C, de préférence de 10 °C à 50 °C, de façon particulièrement préférée de 15 °C à 45 °C avec obtention de la composition opaque, **caractérisé en ce que** le commposant A), ledit au moins un tensioactif,
est choisi parmi les sulfosuccinates éventuellement alcoxylés, en particulier éventuellement éthoxylés, ou représente
un mélange de lauryléthersulfate de sodium et/ou sulfosuccinate éventuellement éthoxylé, avec une bétaïne et le composant B), ledit au moins un émulsifiant, est choisi dans le groupe constitué par les alcoxylates ainsi que les esters d'acides gras et les glycosides.

2. Procédé selon la revendication 1, **caractérisé en ce que** dans le composant A) ledit au moins un tensioactif est choisi dans le groupe constitué par les sulfosuccinates dérivés de monoesters, en particulier le Disodium Laureth Sulfosuccinate.

3. Procédé selon la revendication 1 ou 2, **caractérisé en ce que** dans le composant B) ledit au moins un émulsifiant est choisi dans le groupe constitué par les alcools gras éthoxylés ainsi que les esters polyglycériques d'acides gras.

4. Procédé selon au moins l'une des revendications précédentes, **caractérisé en ce que** la composition de départ de l'étape a) du procédé présente à 20 °C un pH de 4 à 7.

5. Procédé selon au moins l'une des revendications précédentes, **caractérisé en ce que** l'étape c) du procédé est effectuée à une vitesse de refroidissement de 0,1 °C/min à 15 °C/min, de préférence de 0,2 °C/min à 10 °C/min, de façon particulièrement préférée de 0,3 °C/min à 5 °C/min.

6. Procédé selon au moins l'une des revendications précédentes, **caractérisé en ce que** dans l'étape c) du procédé le refroidissement est effectué au moins en partie par l'addition d'eau à la composition de départ, de préférence avec obtention d'une composition opaque ayant une teneur en eau de 50 % en poids à 85 % en poids d'eau, les pourcentages en poids se rapportant à la composition totale.

7. Composition opaque pouvant être obtenue par un procédé selon au moins l'une des revendications précédentes.

8. Composition opaque contenant

   A2) 0,5 % en poids à 15 % en poids d'au moins un tensioactif,
   B2) 0,5 % en poids à 15 % en poids d'au moins un émulsifiant,
   C2) 10 % en poids à 30 % en poids de distéarate d'éthylèneglycol,
   D2) 50 % en poids à 84 % en poids d'eau,
   les pourcentages en poids se rapportant à la composition totale, **caractérisé en ce que**
   le composant A2), ledit au moins un tensioactif,
   est choisi parmi les sulfosuccinates éventuellement alcoxylés, en particulier éventuellement éthoxylés, ou représente
   un mélange de lauryléthersulfate de sodium et/ou sulfosuccinate éventuellement éthoxylé, avec une bétaïne et le composant B2), ledit au moins un émulsifiant, est choisi dans le groupe constitué par les alcoxylates ainsi que les esters d'acides gras et les glycosides.

9. Composition opaque selon la revendication 7 ou 8, **caractérisé en ce qu'**elle présente à une concentration de 0,3 % en poids dans de l'eau, les pourcentages en poids se rapportant à la somme d'eau et de formulation totale, un indice de turbidité de 500 ou plus de 500 Formazine Nephelometrie Units.

10. Procédé pour la préparation de formulations opaques, comprenant les étapes de procédé

   a) fourniture d'une composition de départ comprenant

      A) 0,5 partie en poids à 15 parties en poids d'au moins un tensioactif,
      B) 0,5 partie en poids à 15 parties en poids d'au moins un émulsifiant,
      C) 10 parties en poids à 30 parties en poids de distéarate d'éthylèneglycol,
      D) 20 parties en poids à 85 parties en poids d'eau,

b) agitation de la composition de départ à une température dans une plage de 60 °C à 100 °C, de préférence de 65 °C à 95 °C, de façon particulièrement préférée de 70 °C à 90 °C,

c) refroidissement jusqu'à une température dans une plage de 5 °C à 55 °C, de préférence de 10 °C à 50 °C, de façon particulièrement préférée de 15 °C à 45 °C avec obtention de la composition opaque,

d) mélange de la composition opaque avec des composants supplémentaires dans une plage de température de 5 °C à 40 °C, de préférence de 10 °C à 35 °C, de façon particulièrement préférée de 15 °C à 25 °C, avec obtention d'une formulation opaque, **caractérisé en ce que** le commposant A), ledit au moins un tensioactif, est choisi parmi les sulfosuccinates éventuellement alcoxylés, en particulier éventuellement éthoxylés, ou représente

un mélange de lauryléthersulfate de sodium et/ou sulfosuccinate éventuellement éthoxylé, avec une bétaïne et le composant B), ledit au moins un émulsifiant, est choisi dans le groupe constitué par les alcoxylates ainsi que les esters d'acides gras et les glycosides.

11. Formulation opaque pouvant être obtenue par un procédé selon la revendication 10.

12. Formulation opaque contenant une composition opaque selon au moins l'une des revendications 7 à 9, de préférence en une quantité de 0,1 % en poids à 15 % en poids, les pourcentages en poids se rapportant à la composition totale.

13. Formulation opaque selon la revendication 11 ou 12, **caractérisé en ce qu'**elle présente un indice de turbidité de 600 ou plus de 600 Formazine Nephelometrie Units.

14. Formulation opaque selon au moins l'une des revendications 11 à 13, **caractérisé en ce qu'**elle ne possède pas de propriétés nacrées.

15. Utilisation d'une composition opaque selon au moins l'une des revendications 7 à 9 en tant qu'opacifiant.

| | |
|---|---|
| 0,1% | 363,5 / 240,3 |
| 0,3% | 1103,5 / 520,5 |
| 0,5% | 1863,8 / 710,3 |
| 0,7% | 2548 / 1067,3 |
| 0,8% | 3239,5 / 1126,3 |
| 0,9% | 3467,8 / 1254,8 |
| 1,0% | 3860,8 / 1400 |

0      500     1000    1500    2000    2500    3000    3500    4000    4500

■ 1-7   □ V1-V7

Abbildung 1: Trübungswerte der erfindungsgemäßen Formulierungen 1-7 und Vergleichsformulierung V1-V7

0,1% | 211,5 | 287,8
0,3% | 567,5 | 644
0,5% | 1003,5 | 1094,3
0,7% | 1497,3 | 1518
0,8% | 1718 | 1731,3
0,9% | 1934 | 2083,5
1,0% | 2219,3 | 2427,3

0    500   1000   1500   2000   2500   3000   3500   4000   4500

■ 8-14   □ V8-V14

Abbildung 2: Trübungswerte der in Wasser verdünnten erfindungsgemäßen opaken Zusammensetzung nach Beispiel 1 (8-14) und des Marktstandards Styrene/Acrylates Copolymer (V8-V14).

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 192816613
- JP 2003055165 B